(19) Europäisches Patentamt / European Patent Office / Office européen des brevets

(11) **EP 1 923 095 B1**

(12) **EUROPÄISCHE PATENTSCHRIFT**

(45) Veröffentlichungstag und Bekanntmachung des Hinweises auf die Patenterteilung:
**07.05.2014   Patentblatt 2014/19**

(51) Int Cl.:
**A61N 1/16** *(2006.01)*        **A61N 1/05** *(2006.01)*

(21) Anmeldenummer: **07022251.8**

(22) Anmeldetag: **16.11.2007**

(54) **Elektrode zu Interventionszwecken**

Electrode for intervention purposes

Electrode destinée à des interventions

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **17.11.2006   DE 102006054620**
**12.05.2007   DE 102007022333**

(43) Veröffentlichungstag der Anmeldung:
**21.05.2008   Patentblatt 2008/21**

(73) Patentinhaber: **Biotronik CRM Patent AG**
**6341 Baar (CH)**

(72) Erfinder:
• **Flach, Erhard**
**12305 Berlin (DE)**

• **Geistert, Wolfgang, Dr.**
**79618 Rheinfelden (DE)**
• **Maxfield, Michelle**
**10967 Berlin (DE)**
• **Weiss, Ingo, Dr.**
**12435 Berlin (DE)**
• **Friedrich, Michael, Dr.**
**14532 Kleinmachnow (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
**WO-A-2004/095385        US-A1- 2004 068 313**
**US-A1- 2006 200 218**

EP 1 923 095 B1

**Beschreibung**

Die Erfindung betrifft eine Elektrode zu Interventionszwecken, wie eine Schrittmacher-oder ICD-Elektrode oder ein elektrophysiologischer Katheter, wie sie beispielsweise mit Schrittmachern, Defibrillatoren, Neurostimulatoren und bei Prozeduren der Elektrophysiologie (EP) eingesetzt werden.

Derartige Elektroden weisen bekanntermaßen einen lang gestreckten Elektrodenkörper mit einem distalen und einem proximalen Ende auf. Im Bereich des distalen Endes ist mindestens ein aktiver Elektrodenpol zur Abgabe eines Interventionspulses vorgesehen. Dieser Elektrodenpol ist beispielsweise als direkt am distalen Ende angeordneter Tip-Elektrodenpol, als mit Abstand davon platzierter Ringpol oder als Schockelektrode ausgelegt. Die darüber abgegebenen Interventionspulse sind beispielsweise die Schrittmacherpulse eines Herzschrittmachers bzw. Neurostimulators, ein Hochspannungspuls im Falle eines Defibrillators oder ein Ablationsenergiepuls im Falle eines Ablationsgerätes.

Im Elektrodenkörper verläuft eine Zuleitung umfassend ein erstes Material, welche eine Verbindung zu diesem Elektrodenpol herstellt. Ferner weisen solche Elektroden zu Interventionszwecken meist weitere, allgemein so zu bezeichnende Elektrodenpole auf, mit denen die Elektrode mit Gewebe in Kontakt treten kann. Als Beispiel sind ein Ring-Elektrodenpol einer bipolaren Elektrode oder eines EP-Katheters zu nennen. Ferner ist eine die mindestens eine Zuleitung umgebende Elektrodenummantelung im Elektrodenkörper vorgesehen, welche ein zweites Material umfasst. Meist ist dieses Material isolierend und/oder vom ersten Material verschieden.

In den letzten Jahren haben nun Magnetresonanz-(MR)-Diagnosegeräte, im Folgenden auch MR-Geräte (zum Beispiel Magnet-Resonanz-Tomographen = MRT) genannt, wegen ihrer patientenschonenden, nicht-invasiven und völlig schmerz- sowie nebenwirkungsfreien Untersuchungsmethodik erheblich an Bedeutung gewonnen. Übliche Elektroden zu Interventionszwecken zeigen dabei die Problematik, dass sich derartige Elektroden in Magnetresonanz-Diagnosegeräten unter dem Einfluss der davon generierten elektromagnetischen Strahlung aufgrund elektromagnetischer Induktion und der Abgabe der induzierten Energie im Bereich ihrer Kontaktfläche(n) zum Gewebe stark erwärmen. Der Grund hierfür liegt insbesondere in den massiven, metallischen Zuleitungen zu den Elektrodenpolen, die als Antenne wirken und bei denen aufgrund ihrer Isolierung die durch Hochfrequenz-(HF)-Felder induzierten Antennenströme nur an den Elektrodenpolen, die die elektrische Grenzfläche zum Gewebe bilden, in den Körperelektrolyten abgeleitet werden. Die erwähnten HF-Felder arbeiten beispielsweise in einem Arbeitsfrequenzbereich von 21 MHz bei einem 0,5-Tesla-MR-Tomographen. Der Arbeitsfrequenzbereich kann nach dem derzeitigen Stand der Technik bis zu 300 MHz bei 7-Tesla-MR-Tomographen reichen und liegt typischerweise beispielsweise bei 64MHz bei einem 1,5-Tesla-MR-Tomographen. Da eine extrem starke Erhitzung des Gewebes in der Nähe der Elektrodenpole auftreten kann, ist Trägern von kardiologischen und neurologischen Interventionsgeräten, wie Herzschrittmachern, Neurostimulatoren oder Defibrillatoren, der Zugang zu Magnetresonanz-Diagnosegeräten in aller Regel versperrt.

Um die gefährliche Erwärmung der Körperzellen zu verhindern bzw. zu minimieren, muss der maximale Antennenstrom limitiert bzw. reduziert werden. Bekannte Lösungen schlagen hierzu diskrete Bauelemente vor, welche als Bandsperre oder als Tiefpassfilter wirken und so für die interessierenden Frequenzen den Leitungswiderstand der Antenne limitieren. Andere Lösungen schlagen Kondensatoren vor, die parallel zur Isolation geschaltet sind und so den Antennenstrom ableiten.

Hierzu seien beispielsweise die US 6,944,489, die US 2003/0144720 A1, die US 2003/0144721 A1, die US 2005/0288751 A1 (und die im Wesentlichen Gleichlautenden, gleichzeitig veröffentlichten Parallel-Schriften US 2005/0288752 A1, US 2005/0288754 A1 und US 2005/0288756 A1 ) angeführt.

Prinzipiell besteht diese Möglichkeit, durch bauliche Maßnahmen auf die Induktivität und kapazitive Ankopplung der Antenne Einfluss zu nehmen und damit das Fließen des Antennenstromes zu vermindern, selbigen abzuleiten oder die Resonanzfrequenz zu verschieben. Die aus therapeutischer Sicht gestellten, baulichen Anforderungen an die Elektrode lassen hierzu jedoch nur wenig Spielraum.

Des Weiteren haben Antennen im Gegensatz zu der hier herangezogenen sehr vereinfachten Betrachtung auch weitere Resonanzfrequenzen, so dass die Verschiebung des Resonanzverhaltens der Elektrode dann ggf. bei einem MR-Gerät mit anderen HF-Frequenzen die Resonanzbedingung wiederum erfüllt. Dieser Weg ist daher nicht vorteilhaft.

Als technologischer Hintergrund ist ferner die EP 0 884 024 B1 zu nennen, bei der ein Kondensator zwischen die Zuleitungen für den Ablationspol eines Ablationskatheters und einen ebenfalls daran angeordneten Messpol zur Aufnahme von EKG-Signalen geschaltet ist. Aufgrund dieses Kondensators kann sowohl Hochfrequenzenergie zur Ablation über die Ablationselektrode abgegeben werden als auch gleichzeitig eine EKG-Signalaufnahme erfolgen.

Die US 2006/0009819 A1 offenbart einen Herzschrittmacher mit einer lang gestreckten Elektrode, die mit einem Pulsgenerator-Konnektor verbunden ist. Dabei ist ein passiver Verlustschaltkreis vorgesehen, der elektrisch zwischen einem distalen Abschnitt der Elektrodenzuleitung und einer demgegenüber HF geerdeten Oberfläche geschaltet ist. Der passive Verlustschaltkreis weist eine HF-Impedanz auf, die etwa gleich einer charakteristischen Impedanz der Elektrode in ihrem Implantationszustand im Körper ist. Dadurch wird an den Klemmen des Verlustschaltkreises die Reflexion einfallender Wellen minimiert und deren Energie gezielt hier dissipiert. Der passive Verlustschaltkreis wirkt weiterhin als Tiefpass-Filter, wodurch die Elektrode im Normalbetrieb des Herzschrittmachers funktionsfähig ist.

Ein weiteres Beispiel für eine solche Elektrode ist in US 2006/0200218 A1 dargestellt. Darin wird eine Lösung vorgeschlagen, bei der die Elektrodenummantelung zumindest abschnittsweise frequenzabhängig isolierendes Material mit einer bei niedrigeren Frequenzen geringeren Leitfähigkeit umfasst, während die zumindest eine Zuleitung in bekannter Weise hergestellt ist und aus herkömmlichem Material besteht. Die Frequenzabhängigkeit der Elektrodenummantelung wird durch ein Polymerkomposit realisiert, das dielektrische Eigenschaften aufweist. Die dielektrische Eigenschaft wird durch die Einbettung von dielektrischen Partikeln in eine Polymermatrix hergestellt. So wird eine kapazitive Kopplung der Elektrodenzuleitung zum äußeren Medium ermöglicht. Zu wünschen lässt diese kapazitive Kopplung jedoch eine hohe Effektivität. Denn der resistive frequenzunabhängige Anteil einer solchen kapazitiven Kopplung ist konstant, weshalb die Gesamtimpedanz beim HF-Einfall einer elektromagnetischen Welle nicht beliebig kleine Werte annehmen kann.

Das Dokument WO-A-2004/095 385 offenbart den nächstliegenden Stand der Technik.

Ausgehend von der eingangs geschilderten Problematik liegt der Erfindung die Aufgabe zugrunde, eine Elektrode zu Interventionszwecken so weiterzubilden, dass sie auf konstruktiv einfache Weise auch in Strahlungsfeldern von MR-Diagnosegeräten ohne relevantes Risiko für den Träger platzierbar sind. Insbesondere sollen einfach und kostengünstig herstellbare Materialien genutzt werden, die eine gezielte effektive und ausreichende Verschlechterung der Eigenschaften der Elektrode hinsichtlich ihrer Antennencharakteristik ermöglichen, ohne dass es zu Stromkonzentrationen und entsprechend übermäßigen Erwärmungen an Elektrodenpolen, insbesondere um die Spitze der Elektrode herum, kommen kann.

Diese Aufgabe wird laut Kennzeichnungsteil des Patentanspruches 1 dadurch gelöst, dass das erste und/oder zweite Material so hergerichtet ist, dass das erste Material in einer Polymermatrix eingebettete, leitfähige Partikel enthält, wobei die Konzentration leitfähiger Partikel in der Polymermatrix so eingestellt ist, dass sie soviel größer als die Perkolationsschwelle ist, dass sich der Leitungswiderstand (R) bei zunehmender Frequenz einer einfallenden elektromagnetischen Welle erhöht, und dass die im zweiten Material eingebetteten leitfähigen Partikel eine Konzentration in der Polymermatrix aufweisen, die gleich oder in der Nähe der Perkolationsschwelle ist, damit der Ummantelungswiderstand (r) bei zunehmender Frequenz einer einfallenden elektromagnetischen Welle erniedrigt wird.. Bevorzugt kann es sich dabei um jeweils verschiedene Materialien und/oder verschiedene Konzentrationen handeln, da sich die Perkolationsschwelle bei verschiedenen Materialien individuell einstellt. Es wurde nämlich überraschend festgestellt, dass auch der reelle Anteil, d. h. der ohmsche Anteil, der in der Regel komplexen Leitfähigkeit bei veränderlicher, einfallender Frequenz einer Änderung unterliegt. Dieser Effekt wurde bei einem Materialkomposit aus einem elektrisch leitenden Kunststoff mit Kohlenstoff-Nanoröhren (Carbon Nanotubes = CNT) untersucht und wird vor allem um die sogenannte Perkolationsschwelle herum sichtbar. Ebenfalls überraschend nimmt die ohmsche Leitfähigkeit auch für das reine, d. h. nicht mit Carbon Nanotubes (CNT) dotierte Polymer (untersucht wurde reines Polycarbonat) mit der Frequenz zu, für die hier gestellten Anforderungen ist der Effekt jedoch noch zu gering.

Vorteilhaft ist, dass lösungsgemäß auf einfache Weise und mit einfach herzustellenden Materialien die Eigenschaften so effektiv ausgenutzt werden können, dass die Wirkung von Strahlungsfeldern von Magnetresonanz-Diagnosegeräten auf die Elektrode fast komplett kompensiert werden kann.

Als weitere Aufgabe wird angesehen, ein Herstellungsverfahren für eine erfindungsgemäße Elektrode zu Interventionszwecken zu schaffen, welches eine sichere MR-Kompatibilität der Leitung bietet.

Diese Aufgabe wird durch ein Verfahren gemäß Anspruch 18 gelöst, bei dem eine Zuleitungsummantelung mit Lumen bereitgestellt wird und anschließend eine Zuleitung in das Lumen installiert. In einer bevorzugten Ausgestaltung umfasst das Verfahren den dritten Schritt des luftdichten Eingießens der Zuleitung im durchgängigen Lumen mittels einer leitfähigen Flüssigkeit.

Wie aus der späteren Schilderung des Hintergrundes der Erfindung im Rahmen der Beschreibung von Ausführungsbeispielen deutlich wird, wird erfindungsgemäß die Güte des Schwingkreises, den die Elektrode mit dem Körper zusammen als Antenne bildet, soweit reduziert, dass einerseits die von der Antenne aufgenommene Energie vermindert wird und dass sich andererseits die Verluste in dem Gesamtgebilde Elektrodenleiter/Isolation/Körper so verteilen, dass es zu keiner übermäßigen Stromkonzentration an bestimmten Punkten kommt. Dies kann durch Erhöhung des Leitungswiderstandes R der Zuleitung, durch Verringerung ihres Ummantelungswiderstandes r oder durch beides erreicht werden.

Der wesentliche Vorteil der Verringerung der Güte der Antenne gegenüber einem Bandfilter besteht darin, dass die Wirkweise von der Resonanzfrequenz der Antenne und von der Frequenz des einstrahlenden elektromagnetischen Wechselfeldes unabhängig ist, d. h., die von der Antenne aufgenommene Energie wird bei allen Frequenzen um den Gütefaktor vermindert. Auf diese Weise wirkt die erfindungsgemäße Lösung bei verschiedenen MR-Geräten mit verschiedenen Arbeitsfrequenzen gleichermaßen.

In den abhängigen Ansprüchen sind bevorzugte Ausführungsformen der Elektrode in unterschiedlichen Varianten angegeben. Deren Merkmale, Einzelheiten und Vorteile ergeben sich aus der nachfolgenden Beschreibung, in der Ausführungsbeispiele des Erfindungsgegenstandes anhand der beigefügten Zeichnungen näher erläutert werden. Es zeigen:

Fig. 1        ein schematisches Schaubild zur Darstellung des vereinfachten Funktionsprinzips einer Antenne als offener Schwingkreis,

Fig. 2        ein Ersatzschaltbild der Elektrodenzuleitung als Schwingkreis mit ohmschen Verlusten,

Fig. 3        eine schematische Darstellung eines Elektrodenkörpers mit Zuleitung und Elektrodenummantelung in seiner Körperumgebung,

Fig. 4        ein Diagramm zur Darstellung der Abhängigkeit der normierten Schwingkreisgüte von den ohmschen Widerstandsanteilen der Kapazität und Induktivität des Ersatzschaltbild-Schwingkreises gemäß Fig. 3,

Fig. 5a/5b    Schemazeichnung des Aufbaus einer erfindungsgemäßen Elektrode und Ersatzschaltbild zur Beschreibung der elektrischen Materialeigenschaften

Fig. 6a/6b    Diagramm zur Darstellung der Abhängigkeit des ohmschen Anteils der Leitfähigkeit (a) von der Frequenz und (b) von der Konzentration der Leitpartikel (hier Carbon Nanotubes),

Fig. 7        Schemazeichnung eines Herzschrittmachers mit einer Schrittmacherelektrode,

Fig. 8        Schematische Ansicht des distalen Endbereichs einer Elektrode,

Fig. 9a/9b    schematische Darstellung vom Ausschnitt aus medialen Abschnitten verschiedener erfindungsgemäßen Elektroden mit Seilzuleitungen

Fig. 10a/10b  schematische Darstellung vom Ausschnitt aus medialen Abschnitten alternativer verschiedener erfindungsgemäßen Elektroden mit mindestens einem Wendelabschnitt

Fig. 11a/11b  schematische Darstellung vom Ausschnitt aus medialen Abschnitten bipolarer Elektrodenleitungen, und

Fig. 12a/12b  schematische Darstellung optionaler Ausführungen von distalen Tip- oder Ring-Elektrodenpolen verschiedener erfindungsgemäßer Elektrodenleitungen.

Zum Hintergrund der Erfindung sollen erst die folgenden grundsätzlichen Ausführungen zum Schwingungsverhalten von Antennenkreisen gegeben werden:

Befindet sich ein vorzugsweise lang gestreckter elektrischer Leiter im Raum, wirkt dieser Leiter als Antenne für elektromagnetische Strahlung, die den Raum durchflutet. Dabei wandelt die Antenne die Freiraumwelle in eine Leitungswelle um, wodurch Antennenströme zum Fließen kommen. Diese Situation liegt beispielsweise vor, wenn ein Patient mit implantierten Herzschrittmacherelektroden einem elektromagnetischen Wechselfeld ausgesetzt wird. Die Ströme, die in der als Antenne wirkenden Elektrodenzuleitung dabei entstehen, werden an den Kontaktpunkten zum Gewebe in den Körper geleitet und dort im Wesentlichen in Wärme umgewandelt. Je nach Stärke des elektromagnetischen Wechselfeldes kann das Ausmaß der Erwärmung für den Patienten gefährlich sein. Wechselfelder mit für Schrittmacherträger gefährlicher Stärke treten - wie erwähnt - beispielsweise in MR-Tomographen auf.

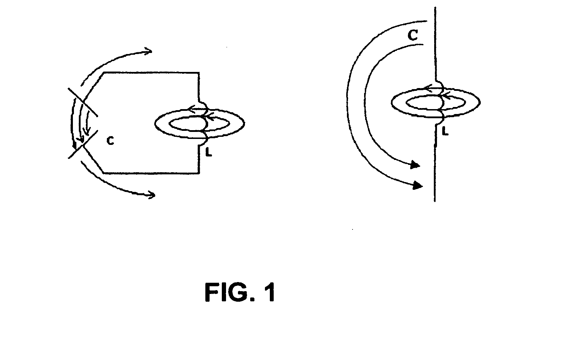

In vereinfachter Darstellung stellt eine Antenne einen offenen Schwingkreis dar, der so verstanden werden kann, als hätte man die Kondensatorplatten eines geschlossenen Schwingkreises in eine lang gestreckte Form auseinander gezogen (Fig. 1). Diese vereinfachte Darstellung beschreibt die Verhaltensweise einer Antenne nur in groben Zügen, sie ist jedoch ausreichend, um das der Erfindung zugrunde liegende Prinzip zu erläutern. In dieser Darstellung verkörpert der Antennenstab A eine Induktivität L, deren Enden kapazitiv miteinander gekoppelt sind (Kapazität C). Der Antennenstrom erreicht seine maximale Amplitude, wenn Resonanz vorliegt, d. h. wenn der Schwingkreis auf der Resonanzfrequenz $f_0$ betrieben wird. Die Resonanzfrequenz des in Fig. 1 dargestellten Schwingkreises ist

$$f_0 = \frac{1}{2\pi\sqrt{LC}}\,.$$

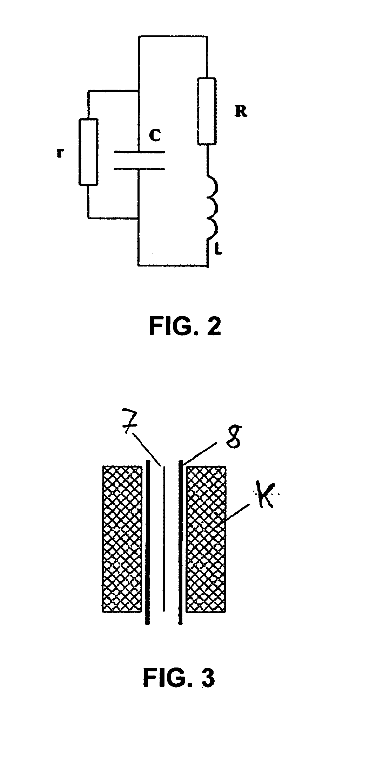

In Realität sind die Komponenten L und C verlustbehaftet, d. h. die Induktivität besitzt einen in Reihe geschalteten ohmschen Widerstand R, während der Kapazität ein ohmscher Widerstand r parallel geschaltet ist (siehe Fig. 2). Im Falle der Schrittmacherelektrode 2, wie in Fig. 3 dargestellt, stellt die Elektrodenzuleitung 7 den Antennenstab dar. Er weist eine von der Geometrie und den Materialeigenschaften abhängige Induktivität L und Leitungswiderstand R auf. Die kapazitive Kopplung der entfernten Leitungsabschnitte erfolgt über das umgebende dielektrische Medium (dazu gehören die Elektrodenisolation 8 und Körper K). Ohmsche Verluste der Isolation und des Körpers sind im Ersatzschaltbild in Fig. 2 durch den Widerstand r zusammengefasst. Für die Funktion als Schrittmacherelektrode ist sowohl für die Stimulation als auch für die Wahrnehmung der Reizantwort (Sensing) anzustreben, dass die Elektrodenzuleitung 7 in Längsrichtung möglichst gut leitet (d. h. R möglichst klein), die Isolation 8 hingegen aber sehr gut ist (d. h. r möglichst groß). Diese ohmschen Komponenten wirken sich auf die Güte Q des Schwingkreises aus, die ein Maß dafür ist, wie hoch die Antennenströme bei gegebener Intensität der elektromagnetischen Strahlung werden können. Die Güte berechnet sich als Quotient

$$Q = \frac{f_0}{B},$$

wobei $f_0$ die Resonanzfrequenz ist und die B die Bandbreite. Die Bandbreite ist durch die Grenzfrequenzen (f=f1, f=f2) definiert, außerhalb derer der Betrag der Leitfähigkeit $|\underline{Y}|$ unter $1/\sqrt{2}$ des Maximalwertes absinkt.

Im Falle des Schwingkreises nach Fig. 2 ist:

$$|\underline{Y}| = \left| \frac{1}{R + j2\pi fL + \dfrac{1}{1/r + j2\pi fC}} \right|, \quad ( j = \sqrt{-1} ).$$

Die Güte des Schwingkreises (und damit auch der maximal mögliche Antennenstrom) ist für bekannte Elektroden besonders gut, d. h. sie steigt mit sinkendem R und steigendem r. Dies wirkt sich besonders im Resonanzfall aus. Bedingt durch die physiologisch erforderte Elektrodenlänge (von ca. 40-60 cm), die dielektrischen Eigenschaften des Körpers und ferner durch die herkömmliche Bauweise tritt die Resonanzbedingung sehr nahe der Larmorfrequenz für Wasserstoff von ca. 64 MHz bei 1,5 T MR-Geräten auf. Mit beispielsweise einer Induktivität von typischerweise 3 μH der Elektrodenzuleitung und einer Kapazität von ca. 2 pF liegt die Resonanzfrequenz des betrachteten Schwingkreises bei fo = 64,97 MHz.

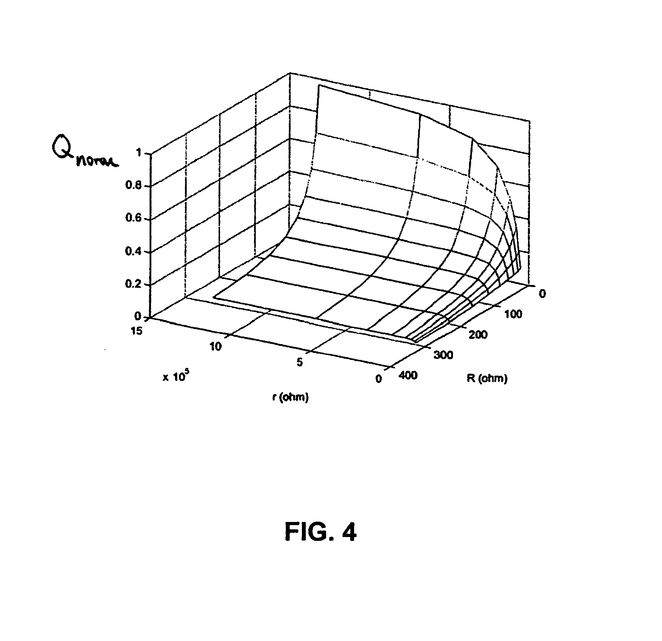

Zur Illustration veranschaulicht Fig. 4 die Abhängigkeit der Güte des verlustbehafteten Schwingkreises nach Fig. 2 von den ohmschen Verlustwiderständen R und r innerhalb praxisrelevanter Wertebereiche wobei L = 3 μH und C = 2 pF angenommen wurde. Qualitativ ist die Gestalt dieser Abhängigkeit auch für weit davon abweichende L und C Werte ähnlich.

Wie in Fig. 4 ersichtlich, kann die Güte der Antenne zweckmäßig verschlechtert werden, indem der Widerstand r der Antennenummantelung vermindert wird, der Widerstand R der Antennenleitung erhöht wird, oder beide Maßnahmen getroffen werden.

Daher besteht die erfindungsgemäße Lösung darin, die Eigenschaften der Elektrode zu verändern, um die Antenneneigenschaften so zu beeinflussen, dass es in Strahlungsfeldern nicht zu Stromkonzentrationen und damit einhergehend zu übermäßigen Erwärmungen an den Elektrodenpolen kommen kann. Die Elektrode umfasst Mittel, welche die Güte des Schwingkreises, den die Elektrode mit dem Körper zusammen als Antenne bildet, soweit reduziert, dass einerseits die von der Antenne aufgenommene Energie vermindert wird und dass sich andererseits die Verluste in dem Gesamtgebilde Elektrodenleiter/Isolation/Körper so verteilen, dass es zu keiner übermäßigen Stromkonzentrationen an bestimmten Punkten kommt.

Nach dem Stand der Technik kann die übliche Elektrodenisolation einerseits durch ein minder-isolierendes Material ersetzt werden, um so den Ummantelungswiderstand der Elektrodenzuleitung zum Körper hin zu reduzieren, die Güte der Antenne zu verschlechtern und die in die Elektrodenzuleitung induzierten Antennenströme und damit die verursachte Erwärmung in Gegenwart elektromagnetischer Wechselfelder zu minimieren. Bei einem vorliegenden Leitungswiderstand einer herkömmlichen Herzschrittmacherelektrodenzuleitung von ca. 50 Ohm und einer Reduktion des Ummantelungswiderstandes (im Sinne des Ersatzschaltbildes aus Fig. 2) von >1 MOhm auf beispielsweise 10 kOhm wird die Güte des Schwingkreises etwa um den Faktor 10 reduziert. Eine beliebige Verkleinerung des Ummantelungswiderstandes ist bei dieser Ausführungsform jedoch nicht möglich, da auch der Stimulationspuls über die nicht mehr ideale Zuleitungsisolation abgeleitet und dadurch gedämpft wird oder umgebendes Gewebe ungewollt stimulieren könnte. Die praktische Grenze hängt vom Leitungswiderstand, vom Gewebewiderstand, von der Gewebeempfindlichkeit und von der Größe des Stimulationspulses ab. Speziell bei Herzschrittmacherelektroden dürfte diese Grenze bei etwa 1 kOhm liegen.

Andererseits kann gemäß des Standes der Technik der Leitungswiderstand der Elektrodenzuleitung erhöht werden. Dabei ist zu beachten, dass der Leitungswiderstand nicht zu stark erhöht wird, da dies die Eigenschaften als Elektrode verschlechtert und die Funktion der Elektrode gefährdet. Die therapeutische Wirksamkeit würde also durch die gestiegenen Verluste innerhalb der Elektrode beeinträchtigt. Übliche Herzschrittmacherelektroden haben Leitungswiderstände bis ca. 100 Ohm. Eine Erhöhung auf ca. 200 Ohm ist aber therapeutisch noch tolerierbar. ICD Elektroden dürfen einen Leitungswiderstand von wenigen Ohm (im einstelligen Ohm-Bereich) nicht überschreiten, daher ist dieser Lösungsansatz dafür nicht oder kaum anwendbar.

Um die Effektivität zu steigern, können die beiden soeben beschriebenen Lösungsansätze optimal kombiniert, d. h. jeweils sowohl der Ummantelungswiderstand des Elektrodenmantels verringert als auch der Leitungswiderstand der Elektrodenzuleitung erhöht werden, so dass gemäß Fig. 4 die Güte bestmöglich verringert wird. Besonders geeignet ist dieser Ansatz für Herzschrittmacher- und Neurostimulationselektroden, wie dies schon aus US 2006/0200218 A1 bekannt ist.

Der Nachteil dieser Lösung gemäß US 2006/0200218A1 ist jedoch, dass die Optimierung und Steigerung der Effektivität nur bedingt möglich ist, da der Ummantelungswiderstand und der Leitungswiderstand fest sind. D. h.: wird der Ummantelungswiderstand niedrig und der Leitungswiderstand hoch eingestellt, um die Erwärmung der Leitung unter HF-Strahlung eines MR-Gerätes zu verhindern, stimuliert die Elektrode nicht mehr richtig. Der hohe Leitungswiderstand R verhindert, dass genügend Stimulationsenergie das Gewebe erreicht. Durch den niedrigen Ummantelungswiderstand fließt Stimulationsenergie ab und erreicht das zu stimulierende Gewebe nicht. Wird aber der Ummantelungswiderstand erhöht und der Leitungswiderstand erniedrigt, um die Therapie nicht zu gefährden, erwärmt sich die Elektrode in einem HF-Feld eines MR-Gerätes zu stark. Somit löst der in US 2006/0200218A1 genannte Gegenstand die Aufgabe nicht.

Zur Lösung der Aufgabe, die Antennengüte zu reduzieren, ist es erfindungsgemäß erforderlich, den Widerstand r der Elektrodenummantelung zu reduzieren und/oder den Leitungswiderstand R zu erhöhen. Da der therapeutische Einsatz nur bei geringen Frequenzen (kleiner 1 MHz) stattfindet und die Antennengüte nur für hohe Frequenzen (größer gleich 1MHz) reduziert werden muss (HF-Strahlung eines MR-Gerätes heute im Bereich 21 MHz bei 0,5-Tesla-MRT's und 300 MHz bei 7-Tesla-MRT's), sind erfindungsgemäß sowohl der Ummantelungswiderstand r und der Leitungswiderstand R der Elektrode frequenzabhängig und zwar dahingehend, dass der Ummantelungswiderstand r mit steigender Frequenz abnimmt beziehungsweise der Leitungswiderstand R mit steigender Frequenz zunimmt. Dies gilt insbesondere für Frequenzen größer 1 MHz.

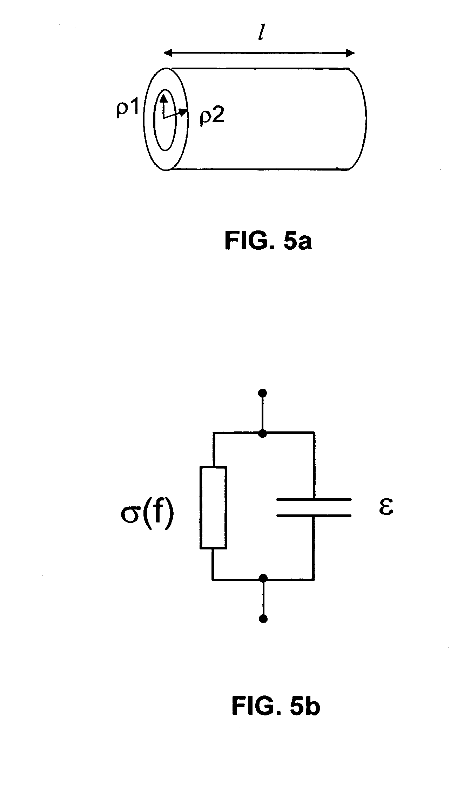

Mit anderen Worten berechnet sich für eine Elektrodenummantelung mit einer Geometrie gemäß Fig. 5a und einem elektrischen Ersatzschaltbild gemäß Fig. 5b der Betrag der Admitanz in radialer Richtung (senkrecht zur Elektrodenlängsachse) ganz allgemein:

$$\left| Y_a(f) \right| = \frac{2\pi l}{\ln\dfrac{\rho_2}{\rho_1}} \sqrt{\sigma_a^2 + (2\pi f)^2 \varepsilon^2} \ .$$

Im Falle des erfindungsgemäß verwendeten Polymerkomposits ist der ohmsche Widerstand selbst frequenzabhängig, d. h. $\sigma_b = \sigma_b(f)$. Damit gilt:

$$\left| Y_b(f) \right| = \frac{2\pi l}{\ln\dfrac{\rho_2}{\rho_1}} \sqrt{\sigma_b^2(f) + (2\pi f)^2 \varepsilon^2} \ .$$

Die Erfindung sieht nun vor, dass das erste Material der Elektrodenzuleitung und das zweite Material der Elektrodenummantelung so ausgewählt und/oder kombiniert sind, dass sich die Widerstandswerte r und R frequenzabhängig verändern. Bei dieser Ausführungsform findet vorzugsweise bei hohen Frequenzen (d. h. bei für MR-Geräte typische Frequenzen) eine Reduktion des Ummantelungswiderstandes r bzw. eine Erhöhung des Leitungswiderstandes R statt.

Dazu werden die Parameter des zweiten Materials - insbesondere die Konzentration leitfähiger Partikel in diesem zweiten Material - und beispielsweise zusätzlich die Dicke der Ummantelung so gewählt, dass sich bei zunehmender Frequenz der Ummantelungswiderstand r erniedrigt. Insbesondere werden die genannten Eigenschaften des zweiten Materials (oder anders genannt des zweiten Materialkomposits) so gewählt, dass sich für Frequenzen unter 1000 Hz ein Ummantelungswiderstand r größer 1 MOhm, für Frequenzen über 5 MHz ein Ummantelungswiderstand r kleiner 10 kOhm und für Frequenzen über 20 MHz ein Ummantelungswiderstand r kleiner 1 kOhm einstellt.

Der Leitungswiderstand R der Elektrodenzuleitung hingegen wird durch Wahl von geeigneten Parametern des ersten Materials - insbesondere die Konzentration leitfähigen Partikel in diesem ersten Material - dahingehend eingestellt, dass sich mit zunehmender Frequenz ein höherer Leitungswiderstandswert R einstellt. Insbesondere stellt sich der Leitungswiderstand R des ersten Materials (oder anders ausgedrückt, des ersten Materialkomposits) so ein, dass er insgesamt für Frequenzen unter 1000 Hz einen Wert von kleiner 100 Ohm und für Frequenzen über 20 MHz einen Wert größer 500 Ohm aufweist. In einer bevorzugten Ausführung steigt der Leitungswiderstand R bei einer Maximalfrequenzen von 300 MHz bei einem 7-Tesla-MRT sogar auf mehr als den 20-fachen Wert von 100 Ohm an, also größer 2 kOhm, bevorzugt größer 4 kOhm an.

Solche frequenzabhängig leitfähige Materialien - wie es sowohl das erste als auch das zweite Material darstellen - kann man herstellen, indem leitfähige Partikel (auch Leitpartikel genannt), insbesondere Nano-Partikel, in eine elektrisch isolierende Matrix eingebettet werden. Diese Partikel sollten eine lang gestreckte Form aufweisen, bevorzugt weisen die leitfähigen Partikel ein Längen- zu Breiten- beziehungsweise Durchmesser-Verhältnis von größer 2, besonders bevorzugt von größer 100 auf. In einer besonderen Ausführung werden dazu Carbon-Partikel mit Dimensionen im ein- bis zweistelligen Nanometerbereich in eine Polymermatrix eingebracht (beispielsweise beschrieben in L.J. Adriaanse, et al.: High-Dilution Carbon-Black/Polymer Composites: Hierachical Percolating Network Derived from Hz to THz ac Conductivity., Physical Review Letters, vol. 78, No. 9, March 1997). Die Frequenzcharakteristik des Materials wird über die Konzentration der leitenden Nanopartikel eingestellt. Weitere Beispiele solcher bevorzugter Leitpartikel sind Carbonfasern mit einem bevorzugten Längen-/Durchmesserverhältnis größer 10, Leitruß, Carbon black, und/oder Metallpartikel. Diese Carbonpartikel sind in einer weiteren bevorzugten Ausführung mit einem Metall oder einer Legierung beschichtet. Als Metall für die Partikel als auch für die Beschichtung sind beispielsweise Aluminium (Al) oder Nickel (Ni) geeignet, bevorzugt werden jedoch biokompatible Metalle, beispielsweise Platin (Pt), Iridium (Ir) oder Gold (Au). Als Legierung kommen bevorzugt solche aus den genannten Metallen in Frage. Eine andere bevorzugte Ausführungsform nutzt frequenzabhängig leitfähige Polymerkomposite statt Carbon/Metallpartikel als Leitzusatz.

Noch effektiver als Carbon-/Metall-Nanopartikel sind Carbon-Nanotubes (CNT). Zur Einstellung einer bestimmten Leitfähigkeitscharakteristik ist eine deutlich kleinere Menge solcher Nanotubes im Vergleich zu Nanopartikeln notwendig.

Wie erwähnt ist insbesondere die Frequenzabhängigkeit des reellen Anteils - anders ausgedrückt, des ohmschen Anteils - der in der Regel komplexen Leitfähigkeit Gegenstand dieser Erfindung.

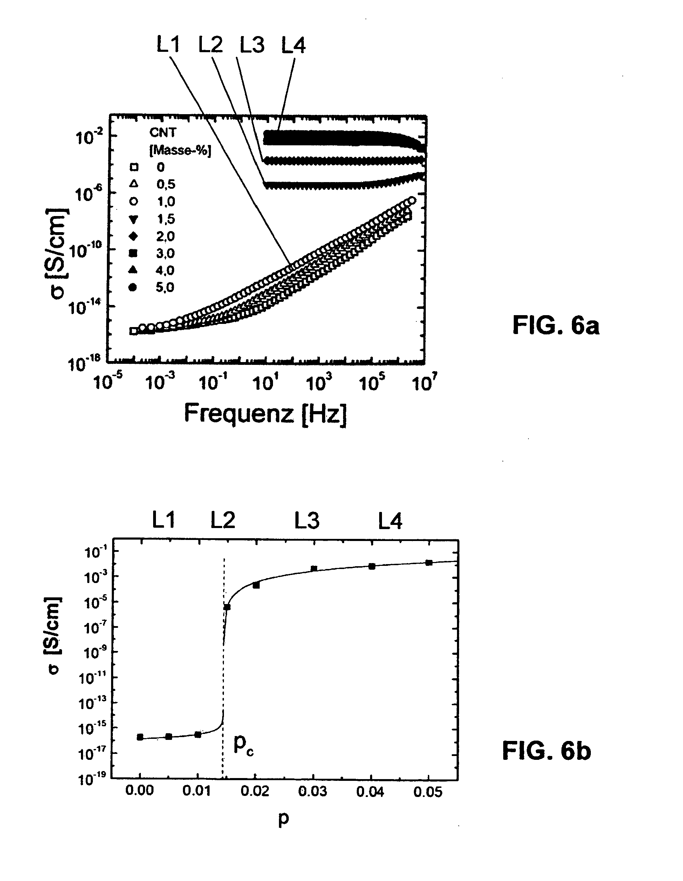

Fig. 6a und 6b zeigen die Abhängigkeit des reellen, d. h. des rein ohmschen Anteils der Leitfähigkeit σ eines Polymerkomposits, das mit Carbon-Nanotubes (CNT) angereichert ist. Überraschend ist, dass die ohmsche Leitfähigkeit auch für das reine d. h. nicht mit Carbon Nanotubes (CNT) dotierte Polymer (untersucht wurde reines Polycarbonat) mit der Frequenz zunimmt. Jedoch ist der Effekt insgesamt für die hier gestellten Anforderungen noch zu gering.

Fig. 6a zeigt die Abhängigkeit der Leitfähigkeit σ von der Frequenz bei verschiedenen Konzentrationen des Leitpartikels CNT im Polymerkomposit. Fig. 6b zeigt Abhängigkeit der Leitfähigkeit σ von der Konzentration p des Leitpartikels CNT bei einer ausgewählten Frequenz.

Bei der Frequenzabhängigkeit des reellen, d. h. des rein ohmschen, Anteils der Leitfähigkeit, werden vier Leitmodi L1 bis L4 unterschieden, die von der Konzentration der Leitpartikel im Polymer bestimmt werden und in Fig. 6a und 6b markiert sind:

- L1: Unterhalb eines bestimmten, Perkolationsschwelle (in Fig. 6b mit "pc" gekennzeichneten) genannten, Konzentrationsschwellwertes befindet sich die Leitfähigkeit generell auf einem niedrigen Niveau und ist kaum von der Leitpartikel-Konzentration abhängig. Sie wächst zwar mit der Frequenz, was aber aufgrund des niedrigen Niveaus für den erfindungsgemäßen Zweck nicht nutzbar ist.
- L2: In Nähe der Perkolationsschwelle ist die Leitfähigkeit gegenüber L1 deutlich höher, insbesondere im niederfrequenten Bereich, so dass die Leitfähigkeit auf einem deutlich höheren Niveau als im Leitmodus L1 liegt. Außerdem nimmt sie mit steigenden Frequenzen zu.
- L3: Bei noch höheren Konzentrationen ist die Leitfähigkeit frequenzunabhängig.
- L4: Wird die Leitpartikeln-Konzentration weiter gesteigert, kehrt sich die Frequenzabhängigkeit um, d. h. die bei niedrigen Frequenzen sehr hohe Leitfähigkeit nimmt zu hohen Frequenzen hin ab.

In einer bevorzugten Ausführung der erfindungsgemäßen Lösung wird das zweite Material der oben beschriebenen Art im Leitmodus L2 zur Realisierung einer Elektrodenummantelung eingesetzt, deren Ummantelungswiderstand r dadurch bei steigender Frequenz sinkt.

In dieser bevorzugten Ausführung wird zusätzlich ein erstes Material der oben beschriebenen Art im Leitmodus L4 zur frequenzabhängen Realisierung mindestens eines Teils der Elektrodenzuleitung eingesetzt, wobei der Leitungswiderstand R der Elektrodenzuleitung durch diesen mindestens einen Teil bei steigender Frequenz ebenfalls steigt. Das elektrische Verhalten dieser Materialien kann somit durch ein Ersatzschaltbild wie in Fig. 5b angegeben, modelliert werden. Der resistive Anteil ist hier durch die Leitfähigkeit σ gekennzeichnet, die erfindungsgemäß und im Gegensatz zu US 2006/0200218 A1 frequenzabhängig ist (σ=σ(f)) und sich je nach eingestelltem Leitmodus mit der Frequenz verändert. Das in US 2006/0200218 A1 vorgeschlagene Material ist für die Realisierung einer Frequenzabhängigkeit (Leitmodus L4) des Leitungswiderstandes R ungeeignet.

Die Erfindung sieht vor, als Polymer vornehmlich Silikon, Polyurethan und/oder Pebax oder ein Silikon-Polyurethan-Copolymer zu verwenden. Bevorzugt sind Typen, die in herkömmlichen Elektrodenummantelungen verwendet werden, und dieses mit den oben beschriebenen, bevorzugten Leitpartikeln zu dotieren.

Ein leitendes Polymerkomposit der erfindungsgemäßen Art ist nicht mit jeder Leitpartikelart herstellbar, zumindest nicht bei Konzentrationen, die unterhalb der Sättigungs- bzw. Verarbeitbarkeitsschwelle liegen. Bei hoch gesättigten Polymerkompositen verändern sich die mechanischen Eigenschaften ungünstig, z.B. wird das Material spröde und ist letztendlich für die Anwendung bei intrakorporalen Elektroden ungeeignet.

Eine Auswahl von leitfähigen Materialien mit den dazugehörigen Konzentrationen, um die Leitmodi 2 oder 4 zu erreichen, sind in der folgenden Tabelle aufgezeigt:

| Leitfähiges Partikel | Leitmodus L2 | Leitmodus L4 |
|---|---|---|
| CNT | 1,25-1,75% | >3% |
| Carbon fibers | 5-10% | >20% |
| Carbon Black: CB | 8-20% | >30% |
| Carbon metallisiert | 10-20% | >25% |

Wie beschrieben befindet sich das erste Material bevorzugt im Leitmodus L4, was bevorzugt bedeutet, dass der Anteil leitfähiger Partikel im ersten Material größer 2 mas%, bevorzugt zwischen 3 und 20 mas% und besonders bevorzugt zwischen 3 und 10 mas% ist.

Andererseits befindet sich wie erwähnt das zweite Material bevorzugt im Leitmodus 2, was bedeutet, dass sich der Anteil leitfähiger Partikel im zweiten Material im Bereich zwischen 1,25 und 20 mas%, bevorzugt zwischen 1,25 bis 10 mas%, besonders bevorzugt zwischen 1,25 bis 1,75 mas% des Gesamtvolumens bewegt.

Für dieses zweite Material im Leitmodus L2 gilt für Frequenzen größer 1 MHz stets $\sigma_b(f_2) > \sigma_b(f_1)$, wenn $f_2 > f_1$. Damit gilt gemäß der weiter oben genannten Formel stets:

$$\left|Y_b\left(f\right)\right| > \left|Y_a\left(f\right)\right|$$

Erfindungsgemäß wird der Außenmantel des Elektrodenkörpers, der bei herkömmlichen Elektroden ein guter Isolator ist (z. B. Silikon oder Polyurethan), aus dem beschriebenen, zweiten Material realisiert, wobei die Elektrodenzuleitung direkten, galvanischen Kontakt zu diesem Material hat. Bei koaxial aufgebauten, mehrpoligen Elektroden besteht dabei in einer Ausführungsform nur der Außenleiter mit einer Außenummantelung aus dem zweiten Material, während die Innenleiter herkömmlich mit einer frequenzunabhängigen Innenummantelung isoliert sind. In weiteren Ausführungsformen ist auch das frequenzunabhängig isolierende Material der Innenummantelung ausgewählter oder aller Innenleiter durch das zweite Material ersetzt. Bei hohen Frequenzen verhält sich so der gesamte Elektrodenkörper wie ein dicker Leiter, der kaum vom Gewebe isoliert ist, d. h., der Ummantelungswiderstand r ist klein, es wird daher bei HF-Einstrahlung weniger Erwärmung verursacht, da die entstehende Antenne eine sehr schlechte Güte besitzt.

In einer weiteren Ausführungsform ist die Kontaktfläche des zweiten Materials zum umgebenden Medium (Körpergewebe bzw. Blut) hin metallisiert (z. B. durch ein Beschichtungs- oder Verdampfungsverfahren) bzw. von einem Metallmantel umgeben (z. B. Ring oder Wendel).

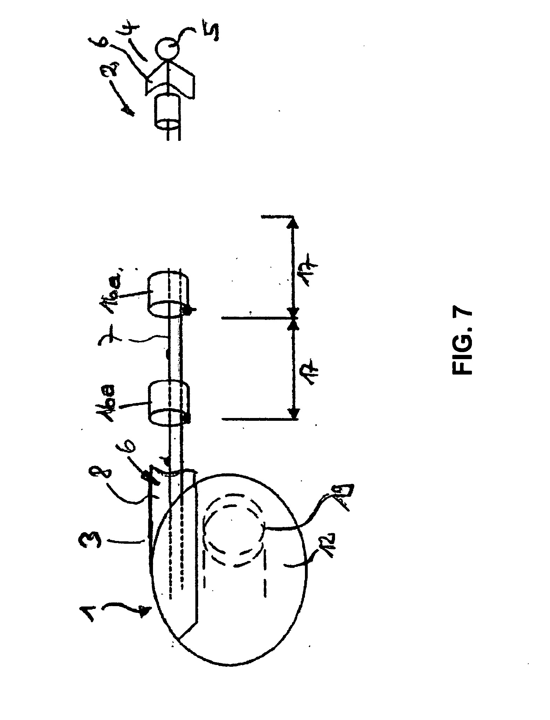

In Fig. 7 ist schematisch ein Herzschrittmacher 1 mit Gehäuse 12 gezeigt, an den in üblicher Weise eine im Herzen platzierte Elektrode 2 mit ihrem proximalen Ende 3 angeschlossen ist. Das distale Ende 4 der Elektrode 2 ist mit einem Tip-Elektrodenpol 5 versehen, über den beispielsweise Stimulationsimpulse an das Herz abgegeben werden. Im lang gestreckten Elektrodenkörper 6 verläuft dazu eine isolierte Zuleitung 7 zum Tip-Elektrodenpol 5.

Es werden dabei nur Abschnitte 16a der Ummantelung 8 und/oder der Zuleitung 7 aus dem ersten und/oder zweiten Material realisiert. In Fig. 7 sind diese Abschnitte als Elektrodenkörpersegmente 16a dargestellt. Hier sind diese als zylinderförmige Segmente realisiert, die vollständig mit dem zweiten Material gefüllt sind. Im Falle einer mehrpoligen Elektrode kontaktiert dieses zweite Material alle oder nur ausgewählte Elektrodenzuleitungen.

In einer weiteren Ausführung werden diese eben beschriebenen Elektrodenkörpersegmente mehrfach in bestimmten Abständen über die Elektrodenzuleitungslänge verteilt, wobei das Kontaktierungsmuster, d. h. die Abschnitte, an denen der oder die Leiter 7 zur Ummantelung aus einem zweiten Material galvanisch in Kontakt stehen, bei allen diesen Elektrodenkörpersegmenten gleich oder unterschiedlich sein können. In einer Variante dieser Ausführung sind die Abstände 17 dieser Elektrodenkörpersegmente alle kleiner als 1/4, bevorzugt als 1/10 der Wellenlänge der HF-Wellen im Arbeitsmedium. Bei dem Arbeitsmedium handelt es sich bevorzugt um einen physiologischen Körper wie einem menschlichen oder tierischen Körper. Beispielsweise kann es sich dabei um umgebendes Körpermedium wie Körpergewebe bzw. Blut handeln.

In einer weiteren nicht gezeigten Ausführung werden unterschiedlich große Abstände 17 und des Weiteren optional unterschiedliche Kontaktierungsmuster dahingehend realisiert, dass die durch die ein erstes und/oder ein zweites Material umfassenden Elektrodenkörpersegmente 16a unterteilten Elektrodenzuleitungsabschnitte hinsichtlich ihrer Wellenimpedanzen so aufeinander abgestimmt werden, dass vorrangig unidirektionale Reflexion an deren Übergängen in Richtung der proximalen Elektrodenseite stattfindet. Hierdurch soll die unerwünschte Leistungsübertragung in Richtung der mit dem Gewebe in Verbindung stehenden Elektrodenpole reduziert werden. Dieses Prinzip ist in Bonmassar, G.: "Resistive Tapered Stri-pline (RTS) in Electroencephalogram Recordings during MRI". IEEE Transactions on Microwave Theory and Techniques, vol. 52. No.8, Aug. 2004 beschrieben.

In einer weiteren nicht gezeigten alternativen Ausführung kann das sich zwischen den in Längsrichtung beabstandeten Elektrodenkörpersegmenten 16a befindliche zweite Material zusätzlich auch abschnittsweise in Radialrichtung ausgebildet sein. Dabei ist ein koaxial um die Längsachse der Elektrode liegender Aufbau von Schichten aus frequenzunabhängig isolierendem Material und zweitem Material vorgesehen. Besonders bevorzugt kann sich die Kombination aus diesen beiden unterschiedlichen Materialien in radialer Richtung beliebig wiederholen.

In einer besonderen Ausführungsform werden die hierfür erforderlichen, nicht homogen verteilten, elektrischen Eigenschaften, d. h. das lokal unterschiedliche Leitfähigkeits- bzw. Frequenzverhalten des Materials durch selektives Dotieren der Polymermatrix mit Leitpartikeln realisiert.

Schließlich ist in Fig. 7 eine Transponderantenne 19 im Herzschrittmacher 1 angedeutet, mit deren Hilfe der Herzschrittmacher 1 von außen parametrisiert bzw. Daten daraus nach außen übertragen werden können. Die Transponderantenne 19 kann nun gleichzeitig zur Detektion einer Hochfrequenz-Strahlung dienen, wie sie im Zuge einer Magnetresonanz-Diagnose beispielsweise in einem MR-Tomographen eingestrahlt wird. Diese Information kann herangezogen werden, um im Falle einer aktiv steuerbaren Auslegung der Koppelelemente diese entsprechend anzusteuern. Die Funktion der Transponderantenne 19 kann auch von der Zuleitung 7 selbst oder dem Gehäuse 12 des Herzschrittmachers 1 wahrgenommen werden.

Damit der vorgesehene Effekt durch die Elektrodenummantelung aus einem zweiten Material besonders gut zur Geltung kommt, muss sichergestellt sein, dass die Zuleitung 7 in einem ständigen Kontakt zu diesem zweiten Material steht. Denn Lufteinschlüsse zwischen der Zuleitung 7 und der Ummantelung wirken als einzelne Isolatoren und machen den gewünschten Effekt zunichte.

Deswegen wird in einer bevorzugten Ausgestaltung der erfindungsgemäßen Elektrodenleitung vorgesehen, die Zuleitung 7 mit einer leitfähigen und aushärtbaren Flüssigkeit in die Lumen in der Zuleitungsisolation 8 einzugießen. So wird sichergestellt, dass es zu keinen Lufteinschlüssen kommt. Die leitfähige Flüssigkeit - bevorzugt auch gelartig - ist bevorzugt ein unter Energieeinwirkung aushärtbares, leitfähiges Polymer.

Das damit verbundene Herstellverfahren umfasst die folgenden Arbeitsschritte:

- Bereitstellen einer Zuleitungsummantelung (8) mit einem vom proximalen Ende bis zu einem am distalen Ende befindlichen Elektrodenpol durchgängigen Lumen,
- Installieren einer Zuleitung in das durchgängige Lumen.

Zusätzlich kann als ein dritter anschließender Schritt das luftdichte Eingießen der Zuleitung im durchgängigen Lumen mittels einer leitfähigen Flüssigkeit.

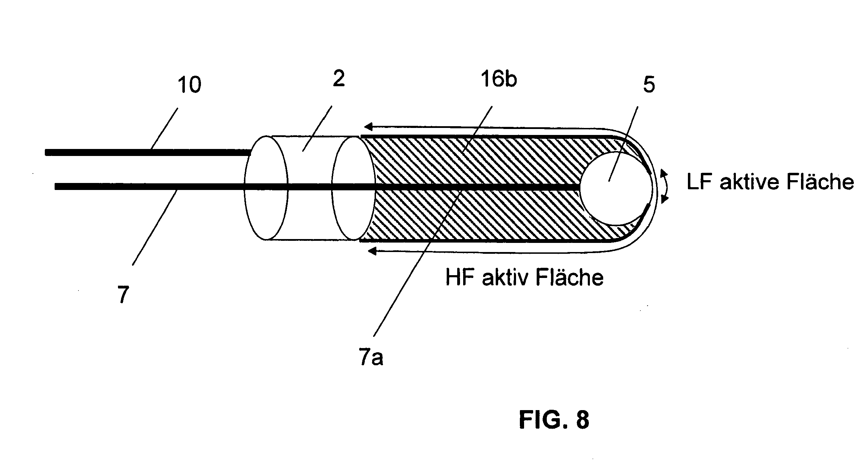

In einem weiteren Ausführungsbeispiel gemäß Fig. 8 wird mindestens ein Elektrodenpol, z. B. der Tip-Elektrodenpol 5, durch das zweite Material 16b, wie in Fig. 8 skizziert, zumindest teilweise bedeckt. Der vorzugsweise große (insbesondere großflächige) Elektrodenkopf wird so bedeckt, dass nur eine kleine Fläche für niederfrequente (LF = Low Frequency) Ströme leitfähig ist ("LF aktive Fläche" in Fig. 8). Stimulationsströme können so mit hoher Stromdichte abgegeben werden. Für hohe Frequenzen (HF = high frequency) hingegen ist nahezu die gesamte Elektrodenkopffläche elektrisch gut leitend ("HF aktive Fläche" in Fig. 8), so dass die Stromdichte und somit die entwickelte spezifische Wärme gering bleiben.

In einer besonderen Ausführung erstreckt sich die aus dem zweiten Material bestehende Elektrodenkopfummantelung 16b bis zum zweiten Pol, wie dem Ring 2 mit Zuleitung 10, einer bipolaren Elektrode. Der Abschnitt 7a der inneren Zuleitung 7, die den Tip-Elektrodenpol 5 kontaktiert, ist dabei gegen das zweite Material der Ummantelung 16b der Ummantelung elektrisch nicht isoliert. Das zweite Material der Ummantelung 16b kann sich auch zwischen zwei Polen einer mehrpoligen Elektrode erstrecken. Auch ein Element 16a wie in Fig. 7 kann sich unmittelbar an den Ring 2 anschließen.

Wie oben beschrieben bewirkt ein mit steigender Frequenz wachsender Leitungswiderstand R der Elektrodenzuleitung ebenfalls eine Verschlechterung der Antennengüte. In einer erfindungsgemäßen Ausführung wird die Elektrodenzuleitung über die gesamte Länge aus einem Material im Leitmodus L4 realisiert. In einer weiteren Ausführung wird eine herkömmliche Elektrodenzuleitung in einen oder mehrere Abschnitte unterteilt, die mittels des genannten Materials verbunden werden. In einer bevorzugten Ausführung beträgt die Länge dieser Abschnitte weniger als 1/4, bevorzugt als 1/10 der Wellenlänge der eingestrahlten Hochfrequenzfelder im Körpermedium.

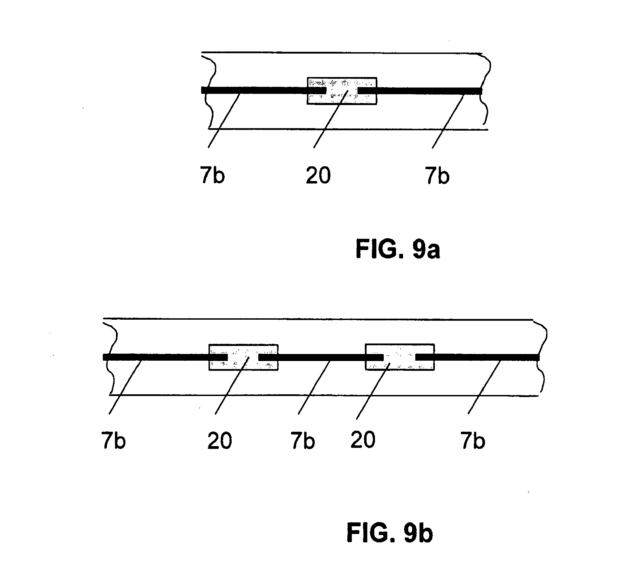

Die Fig. 9a und 9b zeigen schematisch solche Übergangsstellen. Die Abschnitte 20 aus genanntem Material im Leitmodus L4 sind zylindrisch geformt. Seilartige Abschnitte der Elektrodenzuleitung 7b dringen in den Zylinderabschnitt 20 ein und enden in dessen Tiefe. Die Weiterführung der Leitungsabschnitte 7b beginnt bevorzugt in Längsrichtung versetzt, so dass sich die im Zylinderabschnitt 20 befindenden Enden der Leitungsabschnitte 7b nicht berühren. Eine radiale Versetzung der Leitungsabschnitte 7b gegeneinander ist selbstverständlich auch möglich, indem sich entweder eine oder beide Abschnitte 7b, die in den Zylinderabschnitt 20 eindringen, radial von der Längsachse der Elektrodenleitung versetzt sind. Die elektrische Verbindung wird durch den Zylinder 20 aus dem Material im Leitmodus L4 hergestellt. In einer weiteren Ausführung sind die zylindrisch geformten Abschnitte 20 als Röhrchen bzw. Schläuche ausgeführt, durch die zwei aufeinander folgende Leiterabschnitte miteinander verbunden werden. Diese Ausführung ist für einen einfachen Herstellungsprozess besonders günstig. Der Wert des Widerstandes berechnet sich aus der Geometrie dieser Anordnungen sowie der Leitfähigkeit bei einer bestimmten Frequenz.

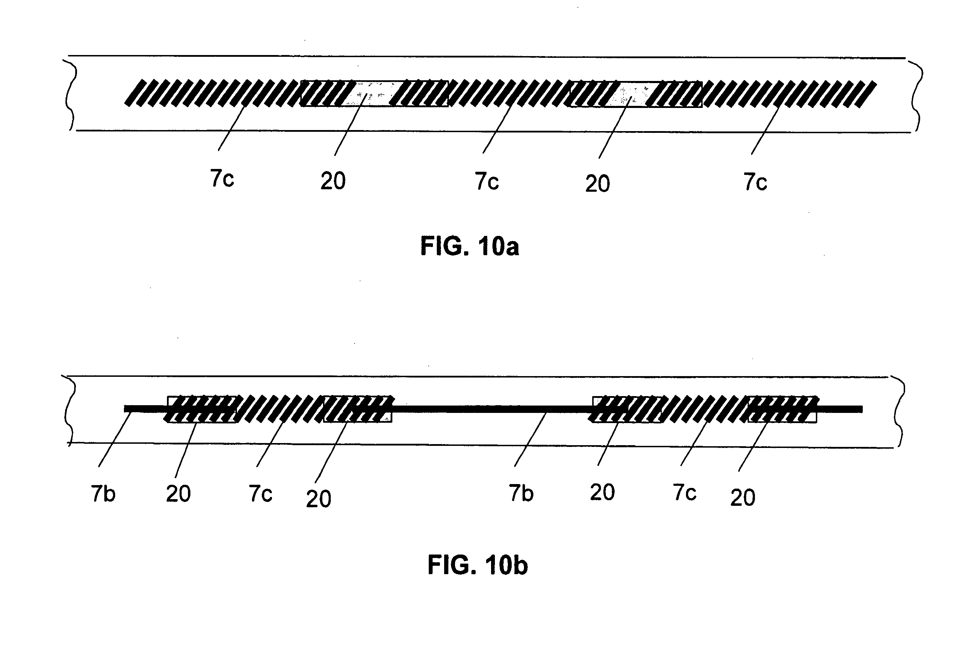

Im Falle einer gewendelten Zuleitung windet sich diese außen über einen Bereich der Mantelfläche dieses Zylinders aus dem erfindungsgemäßen Material. Schematische Beispiele für solche Lösungen sind in Fig. 10a und 10b aufgezeigt. Fig. 10a zeigt gewendelte Zuleitungsabschnitte 7c, welche sich außen über einen Teilbereich eines zylindrisch geformten Abschnitts 20 aus einem Material im Leitmodus L4 winden. Die Enden dieser Wendelabschnitte 7c können so geformt sein, dass sie radial nach innen dringen und so in der Tiefe des zylindrischen Abschnitts 20 enden. Ein einfaches "Aufschieben" der Wendelabschnitte 7c ohne gegenseitige Berührung ist jedoch auch ausreichend. Zwei Wendelabschnitte 7c werden - analog zu der Ausführungen mit Seilabschnitten 7b aus Fig. 9a uns 9b - so am Zylinderabschnitt 20 angebracht, dass sich die Enden der Wendelabschnitte 7c nicht berühren. In einer weiteren Ausführung gemäß Fig. 10b ist eine Kombination aus gewendelten Abschnitten 7c (wie zu Fig. 10a beschrieben) und seilartigen Abschnitten 7b (wie zu Fig. 9a und 9b beschrieben) möglich. Somit muss auch in dieser Ausführung sichergestellt sein, dass sich die Enden der Wendelabschnitte 7c und der Seilabschnitte 7b im zylindrischen Abschnitt 20 aus einem Material im Leitmodus L4 nicht berühren. Dies kann beispielsweise dadurch ermöglicht werden, dass die Wendelabschnitte 7c auf den Zylinderabschnitt 20 aufgeschoben werden und der Seilabschnitt 7b im Zylinderabschnitt 20 in der Tiefe endet oder aber bis zum dem, dem Eindringpunkt gegenüberliegenden, Ende des Zylinderabschnittes 20 geführt werden kann. An jedem zylindrischen Abschnitt 20 kann ein Wendelabschnitt 7c und ein Seilabschnitt 7b angebracht sein, optional können aber auch an einem oder mehreren der zylindrischen Abschnitte 20 nur Wendelabschnitte 7c oder Seilabschnitte 7b befestigt sein.

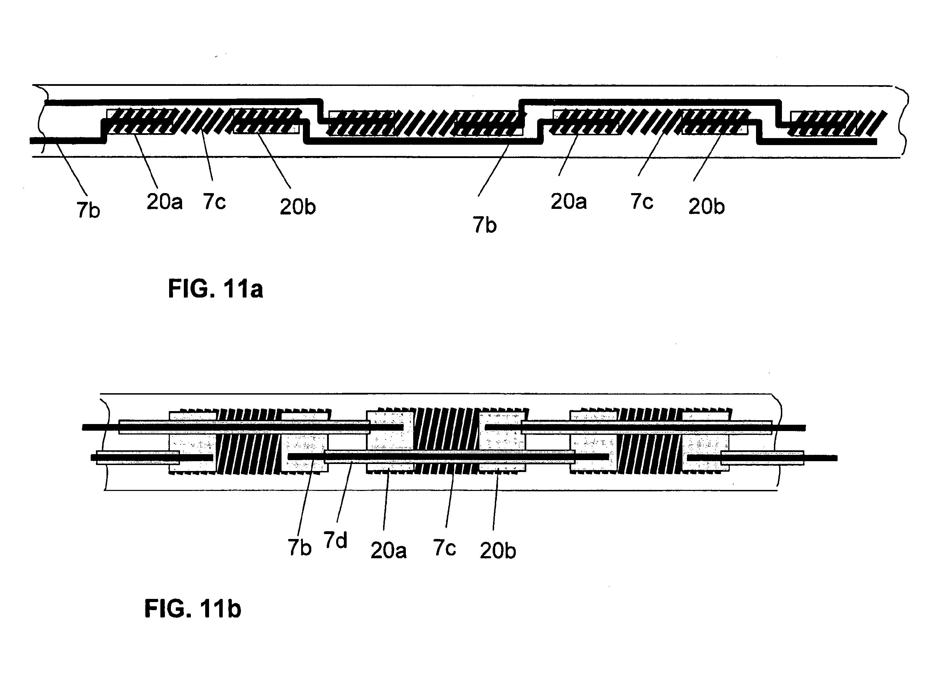

Die soeben genannten Ausführungen lassen sich auch in mehrpoligen Elektrodenleitungen verwirklichen. Fig. 11a zeigt dabei zwei Zuleitungen einer bipolaren Elektrode, die zu zwei verschiedenen Elektrodenpolen führen. Beide Zuleitungen sind dabei so aufgebaut, dass an einem Zylinderabschnitt 20 aus einem Material im Leitmodus L4 jeweils ein Seilabschnitt 7b und ein Wendelabschnitt 7c angebracht sind. Dadurch bilden je zwei Zylinderabschnitte 20a und 20b, die über ein Wendelabschnitt 7c elektrisch verbunden sind, ein "Paar", die dabei auf der Elektrodenachse zu liegen kommen. Die beiden paarweise angeordneten Zylinderabschnitte 20a und 20b und der sie verbindende Wendelabschnitt 7c kommen dabei auf der Elektrodenachse zu liegen. Damit die Zylinderabschnitte 20a und 20b und die Wendelabschnitte 7c auf der Elektrodenachse zu liegen kommen, bilden die Seilabschnitte 7b eine Umgehung, die dadurch gebildet wird, dass der Seilabschnitt 7b nach dem Verlassen des Zylinderabschnittes 20a oder 20b radial nach außen ausgelenkt wird, um am folgenden der andere Zuleitung zugeordneten Zylinderabschnitte 20a oder 20b vorbeigeleitet zu werden, und anschließend wieder in die Elektrodenachse eingelenkt wird, damit eine elektrische Verbindung mit dem diesem Seilabschnitt 7b zugeordneten Zylinderabschnitt 20a oder 20b hergestellt wird.

Um diese Auslenkung der Seilabschnitte 7b zu vermeiden, ist auch ein Aufbau, wie in Fig. 11b gezeigt, möglich. Anstatt die Seilabschnitte 7b an den über einen Wendelabschnitt 7c miteinander verbundenen Zylinderabschnitte 20a und 20b der anderen Zuleitung vorbeizulenken, werden diese Abschnitte dieses Seilabschnittes 7b außen durch beispielsweise einen Schlauch 7d isoliert und durch diese beiden Zylinderabschnitte 20a und 20b und den Wendelabschnitt 7c durchgeführt.

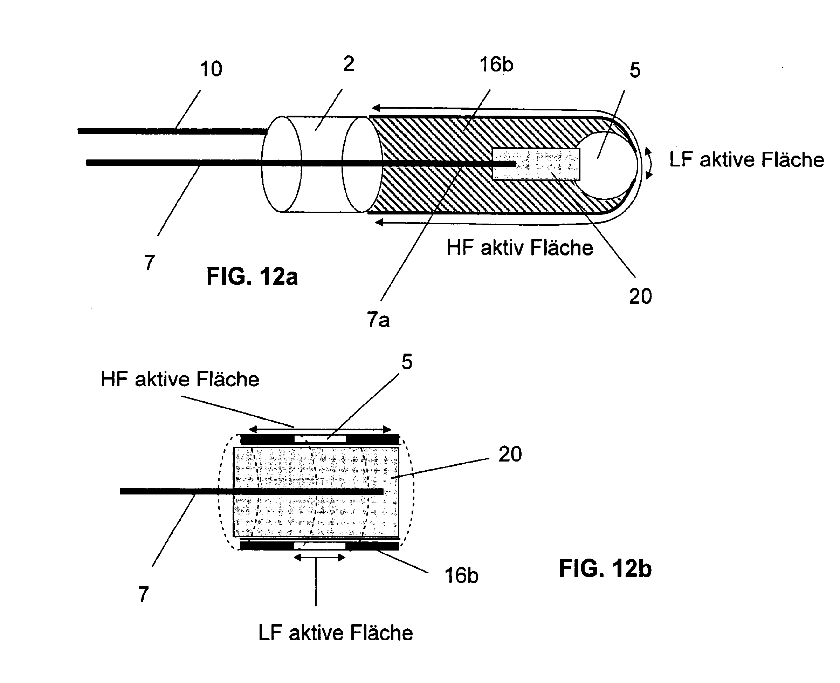

Eine besondere Ausführung sieht vor, den den Leitungswiderstand R realisierenden Leitungsabschnitt in die Konstruktion der Elektrodenspitze zu integrieren. Eine solche Ausführung zeigt Fig. 12a. Darüber hinaus ist der Fall dargestellt, dass auch die erfindungsgemäßen Maßnahmen aus Fig. 8 realisiert sind. Das den Leitungswiderstand R bestimmende Material 20 mit Leitmodus L4 ist direkt von einem Mantel 16b aus Material mit Leitmodus L2 umgeben. Damit ändert sich hier frequenzabhängig der Leitfähigkeitstensor deutlich. Während ein niederfrequenter Strom bevorzugt entlang der Zuleitung geleitet wird, fließt ein hochfrequenter Strom bevorzugt quer (also im rechten Winkel zur Elektrodenzuleitung), er gelangt also nicht oder kaum zum Elektrodenpol 5.

Fig. 12b zeigt eine zusätzliche Form der soeben beschriebenen Konstruktion für eine Stimulationsringelektrode mit einem Elektrodenpol 5. Das Prinzip ist dabei wie soeben beschrieben. Der Elektrodenkörper wird in diesem Bereich durch ein Material 20 im Leitmodus L4 gebildet, welches in geeigneter Weise zumindest teilweise mit einer elektrisch leitenden Metallschicht umhüllt ist. In diesem Beispiel handelt es sich um eine Schicht in Ringform, d. h. der Elektrodenpol 5 wird durch diese Metallschicht gebildet. Die Schicht und/oder das Material 20 direkt neben der Schicht ist teilweise mit einem Material 16b im Leitmodus L2 beschichtet. Die elektrische Zuleitung 7, welche aus Metall oder aber alternativ aus einem Material im Leitmodus 4 besteht, hat keinen direkten Kontakt mit dem Elektrodenpol 5. Der Kontakt erfolgt durch das Material 20 im Leitmodus L4. Während ein niederfrequenter Strom niederohmig von der Zuleitung 7 zum Elektrodenpol 5 geleitet wird, setzt das Material 20 im Leitmodus L4 hochfrequentem Strom einen größeren Widerstand entgegen. Der durch diesen erhöhten Widerstand bereits reduzierte HF-Strom fließt außerdem nicht nur über den Elektrodenpol 5, sondern auch über die gesamte Oberfläche des Materials 16b ab. Die Stromdichte wird entsprechend weiter reduziert.

Eine weitere, hier nicht dargestellte Lösung besteht darin, den den Leitungswiderstand R realisierenden Leitungsabschnitt aus einem ersten Material in einem diesbezüglich modifizierten, herkömmlichen Elektrodenkopf zu integrieren, ohne dass zusätzlich eine Ummantelung aus einem zweiten Material realisiert ist. Eine weitere Ausführungsform sieht vor, dass die in Fig. 12a durch ein zweites Material realisierte Ummantelung stattdessen durch ein Material mit geringer Leitfähigkeit, dafür aber mit hoher Dielektrizitätskonstante realisiert wird. Ein solches Material kann hergestellt werden, indem statt der genannten Leitpartikel z. B. Keramikstäube in die Polymermatrix (im bevorzugten Fall Silikon bzw. Polyurethan) untergemischt werden. In einer besonderen Ausführung umfassen die Partikel Bariumtitanat.

## Patentansprüche

1. Elektrode zu Interventionszwecken, wie Herzschrittmacher-, Neurostimulations-, ICD-Elektrode oder EP-Katheter umfassend:

   - einen lang gestreckten Elektrodenkörper (6) mit einem distalen und einem proximalen Ende (3),
   - mindestens einen Elektrodenpol (5) im Bereich des distalen Endes (4) des Elektrodenkörpers (6) zur Abgabe eines Interventionspulses,
   - mindestens eine im Elektrodenkörper (6) isoliert verlaufende Zuleitung (7) zu dem mindestens einen Elektrodenpol (5), welche ein erstes Material umfasst und
   - eine die mindestens eine Zuleitung (7) umgebende Elektrodenummantelung (8), welche ein zweites Material umfasst, welches in einer Polymermatrix eingebettete leitfähige Partikel enthält,
   **dadurch gekennzeichnet, dass**
   - das erste Material in einer Polymermatrix eingebettete, leitfähige Partikel enthält, wobei die Konzentration leitfähiger Partikel in der Polymermatrix so eingestellt ist, dass sie soviel größer als die Perkolationsschwelle ist, dass sich der Leitungswiderstand (R) bei zunehmender Frequenz einer einfallenden elektromagnetischen Welle erhöht, und
   - die im zweiten Material eingebetteten leitfähigen Partikel ein Konzentration in der Polymermatrix aufweisen, die gleich oder in der Nähe der Perkolationsschwelle ist, damit der Ummantelungswiderstand (r) bei zunehmender Frequenz einer einfallenden elektromagnetischen Welle erniedrigt wird.

2. Elektrode nach Anspruch 1, **dadurch gekennzeichnet, dass** die leitfähigen Partikel eine lang gestreckte Form aufweisen, vorzugsweise mit einem Längen- zu Breitenbeziehungsweise Durchmesser-Verhältnis von größer 2, besonders bevorzugt von größer 100.

3. Elektrode nach einem der Ansprüche 1 oder 2, **dadurch gekennzeichnet, dass** die Polymermatrix vorzugsweise Silikon, Polyurethan und/oder Pebax umfasst und die leitfähigen Partikel Carbon, bevorzugt Carbon black und/oder Carbonfasern, Carbon-Nanopartikel, metallbeschichtete Carbonpartikel und/oder Carbon-Nanotubes, Leitruß und/oder Metallpartikel umfassen.

4. Elektrode nach einem der Ansprüche 1 bis 3, **dadurch gekennzeichnet, dass** bei mehrpoligen Elektroden eine Außenummantelung aus dem zweiten Material und Innenummantelungen für die Zuleitungen aus einem frequenzunabhängigem Isolationsmaterial bestehen.

5. Elektrode nach einem der Ansprüche 1 bis 3, **dadurch gekennzeichnet, dass** bei mehrpoligen Elektroden eine Außenummantelung und eine oder mehrere Innenummantelungen für die Zuleitungen aus dem zweiten Material bestehen.

**6.** Elektrode nach einem der Ansprüche 1 bis 5, **dadurch gekennzeichnet, dass** das erste und/oder zweite Material nur auf Abschnitten (16a, 16b, 20, 20a, 20b) des Elektrodenkörpers (6) und/oder der Zuleitung (7) realisiert ist.

**7.** Elektrode nach Anspruch 6, **dadurch gekennzeichnet, dass** die Abschnitte (16a, 16b, 20, 20a, 20b) aus dem ersten und/oder zweiten Material mehrfach in bestimmten Abständen mit regelmäßigem oder unregelmäßigem Kontaktierungsmuster über den Elektrodenkörper (6) und/oder die Zuleitung (7) verteilt sind.

**8.** Elektrode nach einem der Ansprüche 6 oder 7, **dadurch gekennzeichnet, dass** die Abschnitte (16a, 16b, 20, 20a, 20b) aus dem ersten und/oder zweiten Material in Abständen (17) kleiner als 1/4, vorzugsweise 1/10 der Wellenlänge einer einstrahlenden Hochfrequenz-Welle im Elektrodenkörper (6) und/oder in der Zuleitung (7) angeordnet sind.

**9.** Elektrode nach einem der Ansprüche 6 bis 8, **dadurch gekennzeichnet, dass** aufgrund der Anordnung der Abschnitte (16a, 16b, 20, 20a, 20b) aus dem ersten und/oder zweiten Material und/oder ihres Kontaktierungsmusters und/oder der elektrischen Eigenschaften ihres ersten und/oder zweiten Materials die durch die Abschnitte (16a, 16b, 20, 20a, 20b) unterteilten Teillängen (17) der Elektrodenzuleitung(en) (7a, 7b, 7c) hinsichtlich ihrer Wellenimpedanzen so aufeinander abgestimmt sind, dass eine unidirektionale Reflexion in Richtung des proximalen Elektrodenendes an deren Übergängen stattfindet.

**10.** Elektrode nach einem der Ansprüche 1 bis 9, **dadurch gekennzeichnet, dass** mindestens einer der Elektrodenpole (5) teilweise durch das zweite Material bedeckt ist.

**11.** Elektrode nach einem der Ansprüche 1 bis 10, **dadurch gekennzeichnet, dass** sich die aus dem zweiten Material bestehende Elektrodenummantelung (16b) zwischen zwei Elektrodenpolen (5, 2) einer mehrpoligen Elektrode erstreckt.

**12.** Elektrode nach nach einem der Ansprüche 1 bis 11, **dadurch gekennzeichnet, dass** die Konzentration leitfähiger Partikel im ersten Material so gewählt ist, dass sich für Frequenzen unter 1000 Hz ein Leitungswiderstand (R) kleiner 100 Ohm und für Frequenzen über 20 MHz ein Leitungswiderstand (R) größer 500 Ohm, bevorzugt größer 2 kOhm, besonders bevorzugt 4 kOhm einstellt.

**13.** Elektrode nach Anspruch 12, **dadurch gekennzeichnet, dass** die Konzentration durch den Anteil leitfähiger Partikel im ersten Material bei größer 2 mas% des Gesamtvolumens liegt.

**14.** Elektrode nach nach einem der Ansprüche 1 bis 10, **dadurch gekennzeichnet, dass** der Anteil leitfähiger Partikel im ersten Material der Elektrodenummantelung (8) so gewählt ist und die Materialparameter und die Dicke der Elektrodenummantelung (16a, 16b) so gewählt sind, dass sich für Frequenzen unter 1000 Hz ein Ummantelungswiderstand (r) größer 1 MOhm, für Frequenzen über 5 MHz ein Ummantelungswiderstand (r) kleiner 10 kOhm und für Frequenzen über 20 MHz ein Ummantelungswiderstand (r) kleiner 1 kOhm einstellt.

**15.** Elektrode nach Anspruch 14, **dadurch gekennzeichnet, dass** die die Konzentration durch den Anteil leitfähiger Partikel im zweiten Material zwischen 1,25 und 20 mas% des Gesamtvolumens liegt.

**16.** Verfahren zur Herstellung einer Elektrode nach den Ansprüchen 1 bis 15, umfassend folgende Schritte:

- Bereitstellen einer Elektrodenummantelung (8) gemäß den Ansprüchen 1-15 mit einem vom proximalen Ende bis zu einem am distalen Ende befindlichen Elektrodenpol durchgängigen Lumen und
- Installieren einer Zuleitung (7) gemäß den Ansprüchen 1-15 in das durchgängige Lumen.

**17.** Verfahren nach Anspruch 16, weiter umfassend den Schritt des luftdichten Eingießens der Zuleitung im durchgängigen Lumen mittels einer leitfähigen Flüssigkeit.

**Claims**

**1.** An electrode for interventional purposes such as a cardiac pacemaker, neurostimulation ICD electrode or EP catheter, comprising:

- an elongated electrode body (6) having a distal and a proximal end (3),
- at least one electrode pole (5) in the region of the distal end (4) of the electrode body (6) for delivering an intervention pulse,
- at least one supply line (7) which is insulated within the electrode body (6) and runs to the at least one electrode pole and which comprises a first material, and
- an electrode sheath (8) that encloses the at least one supply line (7) and comprises a second material that contains conductive particles embedded in a polymer matrix,
**characterized in that**
- the first material contains conductive particles embedded in a polymer matrix, wherein the concentration of conductive particles in the polymer matrix is set in such a manner that it is greater than the percolation threshold by such an amount that the line resistance (R) increases with rising frequency of an incident electromagnetic wave, and
- the conductive particles embedded in the second material have a concentration in the polymer matrix that is equal or near the percolation threshold in order that the sheath resistance (r) is reduced with rising frequency of an incident electromagnetic wave.

2. The electrode according to claim 1, **characterized in that** the conductive particles have an elongated shape, preferably with a length to width ratio or diameter ratio of greater than 2, particularly preferred of greater than 100.

3. The electrode according to claim 1 or claim 2, **characterized in that** the polymer matrix preferably comprises silicone, polyurethane and/or polyether block amide, and the conductive particles comprise carbon, preferably carbon black and/or carbon fibers, carbon nanoparticles, metal-coated carbon particles and/or carbon nanotubes, conductive soot and/or metal particles.

4. The electrode according to any one of the claims 1 to 3, **characterized in that** in the case of multipolar electrodes, an external sheath consists of the second material, and internal sheaths for the supply lines consist of a frequency-independent insulation material.

5. The electrode according to any one of the claims 1 to 3, **characterized in that** in the case of multi-polar electrodes, an external sheath and one or more internal sheaths for the supply lines consist of the second material.

6. The electrode according to any one of the claims 1 to 5, **characterized in that** the first and/or the second material is implemented only on sections (16a, 16b, 20, 20a, 20b) of the electrode body (6) and/or the supply line (7).

7. The electrode according to claim 6, **characterized in that** the sections (16a, 16b, 20, 20a, 20b) made of the first and/or second material are distributed multiple times at given intervals with regular or irregular contacting patterns over the electrode body (6) and/or the supply line (7).

8. The electrode according to claim 6 or claim 7, **characterized in that** the sections (16a, 16b, 20, 20a, 20b) made of the first and/or second material are arranged at intervals (17) of less than 1/4, preferably less than 1/10 of the wavelength of an irradiating high-frequency wave in the electrode body (6) and/or in the supply line (7).

9. The electrode according to any one of the claims 6 to 8, **characterized in that** due to the arrangement of the sections (16a, 16b, 20, 20a, 20b) made of the first and/or second material, and/or their contacting patterns, and/or the electric properties of their first and/or second material, the partial lengths (17) of the electrode supply line(s) (7a, 7b, 7c), which are divided by the sections (16a, 16b, 20, 20a, 20b), are attuned to each other with regard to their wave impedances such that a unidirectional reflection in the direction of the proximal electrode end takes place at the transitions of said partial lengths.

10. The electrode according to any one of the claims 1 to 9, **characterized in that** at least one of the electrode poles (5) is partially covered by the second material.

11. The electrode according to any one of the claims 1 to 10, **characterized in that** the electrode sheath (16b) consisting of the second material extends between two electrode poles (5, 2) of a multipolar electrode.

12. The electrode according to any one of the claims 1 to 11, **characterized in that** the concentration of conductive particles in the first material is selected such that for frequencies below 1000 Hz, a line resistance (R) of less than 100 Ohm is obtained, and for frequencies above 20 MHz, a line resistance (R) greater than 500 Ohm, preferably greater than 2kOhm, particularly preferred 4 kOhm is obtained.

13. The electrode according to claim 12, **characterized in that** due to the proportion of conductive particles, the concentration in the first material is greater than 2% by mass based on the total volume.

14. The electrode according to any one of the claims 1 to 10, **characterized in that** the proportion of conductive particles in the first material of the electrode sheath (8) is selected such, and the material parameters and the thickness of the electrode sheath (16a, 16b) are selected such that for frequencies below 1000 Hz, a sheath resistance (r) greater than 1 MOhm is obtained, for frequencies above 5 MHz, a sheath resistance (r) of less than 10 kOhm is obtained, and for frequencies above 20 MHz, a sheath resistance (r) of less than 1 kOhm is obtained.

15. The electrode according to claim 14, **characterized in that** due to the proportion of conductive particles, the concentration in the second material is between 1.25 and 20% by mass based on the total volume.

16. A method for producing an electrode according to the claims 1 to 15, comprising the following steps:

- providing an electrode sheath (8) according to the claims 1 to 15, comprising a continuous lumen extending from the proximal end to an electrode pole located at the distal end, and
- installing a supply line (7) according to the claims 1 to 15 in the continuous lumen.

17. The method according to claim 16, further comprising the step of airtight embedding the supply line in the continuous lumen by means of a conductive liquid.

## Revendications

1. Électrode utilisée à des fins d'intervention, telle qu'une électrode de stimulateur cardiaque, de neurostimulation ou ICD ou un cathéter EP, comprenant:

- un corps d'électrode allongé (6) avec une extrémité distale et une extrémité proximale (3),
- au moins un pôle d'électrode (5) dans la région de l'extrémité distale (4) du corps d'électrode (6), pour la délivrance d'une impulsion d'intervention,
- au moins une ligne d'alimentation (7) s'étendant de façon isolée dans le corps d'électrode (6), vers l'au moins un pôle d'électrode (5), laquelle comprend un premier matériau, et
- une enveloppe d'électrode (8) entourant l'au moins une ligne d'alimentation (7), laquelle comprend un deuxième matériau contenant des particules conductrices enrobées dans une matrice polymère,
**caractérisée en ce que**
- le premier matériau contient des particules conductrices enrobées dans une matrice polymère, la concentration de particules dans la matrice polymère étant définie de manière à dépasser le seuil de percolation au point que la résistivité (R) croît lors d'une augmentation de la fréquence d'une onde électromagnétique incidente, et
- les particules conductrices enrobées dans le deuxième matériau présentent une concentration dans la matrice polymère, laquelle est égale au seuil de percolation ou proche de celui-ci, afin de réduire la résistance de l'enveloppe (r) lors d'une augmentation de la fréquence d'une onde électromagnétique incidente.

2. Électrode selon la revendication 1, **caractérisée en ce que** les particules conductrices présentent une forme allongée, de préférence avec un rapport de longueur sur largeur ou diamètre supérieur à 2, et mieux, supérieur à 100.

3. Électrode selon l'une des revendications 1 ou 2, **caractérisée en ce que** la matrice polymère comprend de préférence du silicone, du polyuréthane et/ou du Pebax, et les particules conductrices comprennent du carbone, de préférence du noir de carbone et/ou des fibres de carbone, des nanoparticules de carbone, des particules de carbone recouvertes de métal et/ou des nanotubes de carbone, du noir conducteur et/ou des particules métalliques.

4. Électrode selon l'une des revendications 1 à 3, **caractérisée en ce qu'**avec des électrodes à plusieurs pôles, une enveloppe extérieure est constituée du deuxième matériau et des enveloppes intérieures pour les lignes d'alimentation sont constituées d'un matériau isolant indépendant de la fréquence.

5. Électrode selon l'une des revendications 1 à 3, **caractérisée en ce qu'**avec des électrodes à plusieurs pôles, une enveloppe extérieure et une ou plusieurs enveloppes intérieures pour les lignes d'alimentation sont constituées du deuxième matériau.

**6.** Électrode selon l'une des revendications 1 à 5, **caractérisée en ce que** le premier et/ou le deuxième matériau n'est utilisé que sur des sections (16a, 16b, 20, 20a, 20b) du corps d'électrode (6) et/ou de la ligne d'alimentation (7).

**7.** Électrode selon la revendication 6, **caractérisée en ce que** les sections (16a, 16b, 20, 20a, 20b) constituées du premier et/ou du deuxième matériau sont réparties par intervalles répétés sur le corps d'électrode (6) et/ou la ligne d'alimentation (7), avec un modèle de mise en contact régulier ou irrégulier.

**8.** Électrode selon l'une des revendications 6 ou 7, **caractérisée en ce que** les sections (16a, 16b, 20, 20a, 20b) constituées du premier et/ou du deuxième matériau sont agencées par intervalles (17) inférieurs à 1/4, de préférence à 1/10 de la longueur d'onde d'une onde haute fréquence incidente, dans le corps d'électrode (6) et/ou dans la ligne d'alimentation (7).

**9.** Électrode selon l'une des revendications 6 à 8, **caractérisée en ce qu'**en raison de l'agencement des sections (16a, 16b, 20, 20a, 20b) constituées du premier et/ou du deuxième matériau, et/ou de leur modèle de mise en contact et/ou des propriétés électriques de leur premier et/ou deuxième matériau, les longueurs partielles (17) de la/des ligne(s) d'alimentation d'électrode(s) (7a, 7b, 7c), divisées par les sections (16a, 16b, 20, 20a, 20b), sont adaptées les unes aux autres quant à leurs impédances d'onde, de sorte qu'une réflexion unidirectionnelle dans la direction de l'extrémité proximale d'électrode a lieu au niveau de leurs transitions.

**10.** Électrode selon l'une des revendications 1 à 9, **caractérisée en ce qu'**au moins l'un des pôles d'électrode (5) est partiellement recouvert par le deuxième matériau.

**11.** Électrode selon l'une des revendications 1 à 10, **caractérisée en ce que** l'enveloppe d'électrode (16b) constituée du deuxième matériau s'étend entre deux pôles d'électrode (5, 2) d'une électrode à plusieurs pôles.

**12.** Électrode selon l'une des revendications 1 à 11, **caractérisée en ce que** la concentration de particules conductrices dans le premier matériau est choisie de manière à ce que pour des fréquences inférieures à 1000 Hz, une résistivité (R) est inférieure à 100 Ohm, et pour des fréquences supérieures à 20 MHz, une résistivité (R) est supérieure à 500 Ohm, de préférence supérieure à 2 kOhm, et mieux, de 4 kOhm.

**13.** Électrode selon la revendication 12, **caractérisée en ce que** la concentration de la part de particules conductrices dans le premier matériau est supérieure à 2% en volume du volume total.

**14.** Électrode selon l'une des revendications 1 à 10, **caractérisée en ce que** la part de particules conductrices dans le premier matériau de l'enveloppe d'électrode (8), ainsi que le paramètre de matériau et l'épaisseur de l'enveloppe d'électrode (16a, 16b), sont choisis de telle façon que pour des fréquences inférieures à 1000 Hz, une résistance d'enveloppe (r) est supérieure à 1 MOhm, pour des fréquences supérieures à 5 MHz, une résistance d'enveloppe (r) est inférieure à 10 kOhm, et pour des fréquences supérieures à 20 MHz, une résistance d'enveloppe (r) est inférieure à 1 kOhm.

**15.** Électrode selon la revendication 14, **caractérisée en ce que** la concentration de la part de particules conductrices dans le deuxième matériau est comprise entre 1,25 et 20% en volume du volume total.

**16.** Procédé pour la fabrication d'une électrode selon les revendications 1 à 15, comprenant les étapes suivantes:

- mise à disposition d'une enveloppe d'électrode (8) selon l'une des revendications 1 à 15, avec un lumen s'étendant en continu à partir de l'extrémité proximale jusqu'à un pôle d'électrode situé à l'extrémité distale, et
- installation d'une ligne d'alimentation (7) selon l'une des revendications 1 à 15 dans le lumen continu.

**17.** Procédé selon la revendication 16, comprenant en outre l'étape de coulage hermétique de la ligne d'alimentation dans le lumen continu au moyen d'un liquide conducteur.

**FIG. 1**

**FIG. 2**

**FIG. 3**

**FIG. 4**

**FIG. 5a**

**FIG. 5b**

FIG. 6a

FIG. 6b

FIG. 7

LF aktive Fläche

5

16b

2

10

HF aktiv Fläche

7a

7

FIG. 8

FIG. 9a

FIG. 9b

FIG. 10a

FIG. 10b

FIG. 11a

FIG. 11b

10     2     16b     5

LF aktive Fläche

HF aktiv Fläche

7

7a

20

**FIG. 12a**

HF aktive Fläche

5

20

7

16b

**FIG. 12b**

LF aktive Fläche

EP 1 923 095 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6944489 B
- US 20030144720 A1
- US 20030144721 A1
- US 20050288751 A1
- US 20050288752 A1
- US 20050288754 A1
- US 20050288756 A1
- EP 0884024 B1
- US 20060009819 A1
- US 20060200218 A1
- WO 2004095385 A

**In der Beschreibung aufgeführte Nicht-Patentliteratur**

- **L.J. ADRIAANSE et al.** High-Dilution Carbon-Black/Polymer Composites: Hierachical Percolating Network Derived from Hz to THz ac Conductivity. *Physical Review Letters,* Marz 1997, vol. 78 (9
- **BONMASSAR, G.** Resistive Tapered Stri-pline (RTS) in Electroencephalogram Recordings during MRI. *IEEE Transactions on Microwave Theory and Techniques,* August 2004, vol. 52 (8